(19) Europäisches Patentamt
European Patent Office
Office européen des brevets

(11) Veröffentlichungsnummer: **0 003 052**
**B1**

(12) # EUROPÄISCHE PATENTSCHRIFT

(45) Veröffentlichungstag der Patentschrift: **18.03.81**

(51) Int. Cl.³: **C 07 D 233/64**

(21) Anmeldenummer: **78101858.5**

(22) Anmeldetag: **28.12.78**

(54) **Verfahren zur Herstellung von 4-Methyl-5-chlormethylimidazol.**

(30) Priorität: **03.01.78 DE 2800148**

(43) Veröffentlichungstag der Anmeldung: **25.07.79 Patentblatt 79/15**

(45) Bekanntmachung des Hinweises auf die Patenterteilung: **18.03.81 Patentblatt 81/11**

(84) Benannte Vertragsstaaten: **CH DE FR GB IT**

(56) Entgegenhaltungen: **GB-A-1 341 376**

(73) Patentinhaber: **BASF Aktiengesellschaft, Carl-Bosch-Strasse 38, D-6700 Ludwigshafen (DE)**

(72) Erfinder: **Halbritter, Klaus, Dr., Schwarzwaldstrasse 19, D-6800 Mannheim 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchgebühr entrichtet worden ist (Art. 99(1) Europäisches Patentübereinkommen).



BUNDESDRUCKEREI BERLIN

# Verfahren zur Herstellung von 4-Methyl-5-chlormethyl-imidazol

Die Erfindung betrifft ein Verfahren zur Herstellung von 4-Methyl-5-chlormethyl-imidazol in Form seines Hydrochlorids durch Chlormethylierung von 4-Methylimidazol.

Das 4-Methyl-5-chlormethyl-imidazol-hydrochlorid wurde bisher hergestellt aus 4-Methyl-5-hydroxymethyl-imidazol durch Umsetzung mit anorganischen Säurehalogeniden, wie es von A. J. Ewins im J. chem. Soc. 99, 2052—2059 (1911) oder G. J. Durant et al im J. med. Chem. 19, 923—928 (1976) beschrieben wird. Nachteilig an diesen Verfahren ist, daß von 4-Methyl-5-hydroxymethyl-imidazol ausgegangen werden muß, da diese Verbindung verhältnismäßig schwer in nur umständlicher Weise hergestellt werden kann. So wird bisher das 4-Methyl-5-hydroxymethyl-imidazol aus 4-Methyl-imidazol-5-carbonsäureester durch Reduktion mit Alkalimetallen oder Calcium in flüssigem Ammoniak hergestellt. Dieses Verfahren ist langwierig, teuer und im technischen Maßstab wegen der zu bewältigenden Mengen Alkalimetall in flüssigem Ammoniak verhältnismäßig schwierig durchzuführen und wird in der DE-OS 26 37 670 beschrieben.

Es wurde nun gefunden, daß man 4-Methyl-5-chlormethyl-imidazol-hydrochlorid auf einfache Weise in großtechnischem Maßstabe herstellen kann, indem man 4-Methylimidazol in wäßirger Lösung mit Formaldehyd oder einem Oligomeren des Formaldehyds in Gegenwart einer überschüssigen Menge Chlorwasserstoff bei Temperaturen von 25 bis 160° C umsetzt.

Die Erfindung wird durch das folgende Formelschema veranschaulicht:

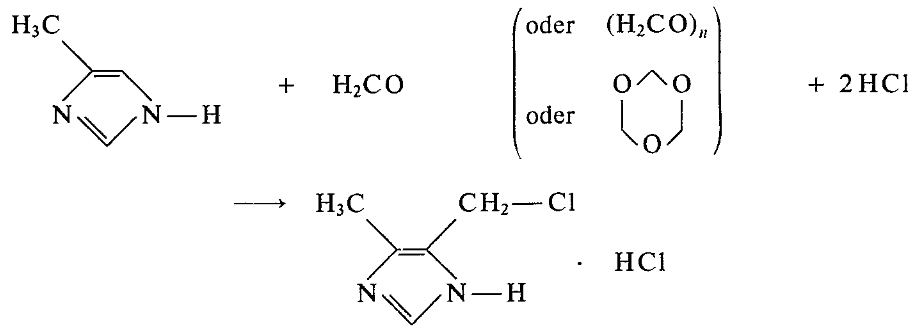


Zweckmäßig wird bei dem erfindungsgemäßen Verfahren daß 4-Methylimidazol in wäßriger konzentrierter Salzsäure gelöst, wobei in der Regel 10 bis 25gewichtsprozentige Lösungen des 4-Methyl-imidazols hergestellt werden.

Der Formaldehyd kann in üblicher wäßriger Lösung, enthaltend 15 bis 40 Gew.-% Formaldehyd, gasförmig oder in Form seiner festen Oligomeren, wie Paraformaldehyd oder 1,3,5-Trioxan, verwendet werden. Das Molverhältnis 4-Methylimidazol zu Formaldehyd liegt vorteilhaft bei 1 : 1 bis 1 : 1,5. Dabei wird auch bei Verwendung von Oligomeren des Formaldehyds auf das Monomere bezogen.

Der Chlorwasserstoff wird in überschüssiger Menge vorteilhafterweise als wäßrige konzentrierte Salzsäure, enthaltend 20 bis 40 Gew.-% Chlorwasserstoff, oder gasförmig verwendet. Bevorzugt wird in einem System wäßrige konzentrierte Salzsäure/gasförmiger Chlorwasserstoff gearbeitet. Daher wird, wenn man die Chlormethylierungsreaktion bei Normaldruck durchführt, vorteilhaft durch die Reaktionsmischung HCl-Gas durchgeleitet, oder, wenn man unter Druck in einem geschlossenen System, z. B. in einem Autoklav oder Kessel, arbeitet, kann gegebenenfalls zusätzlich HCl-Gas bis zu 10 bar aufgepreßt werden.

Die erfindungsgemäße Chlormethylierungsreaktion wird bei Temperaturen von 25 bis 160° C, vorzugsweise bei Temperaturen von 50 bis 110° C, durchgeführt. Gemäß den angewandten Temperaturen stellt sich, wenn man im geschlossenen System arbeitet, der entsprechende Druck ein.

Die Umsetzung ist in Abhängigkeit von den angewandten Temperaturen in der Regel innerhalb eines Zeitraums von 5 bis 20 Stunden beendet. Die vollständige Umsetzung kann spektroskopisch verfolgt werden, beispielsweise durch NMR-Spektroskopie, indem man Proben aus dem Reaktionsgemisch entnimmt und nach dem Eindampfen mit konzentrierter DCl (in $D_2O$) analysiert.

Die Aufarbeitung des Reaktionsprodukts erfolgt in üblicher Weise, beispielsweise durch Abdestillieren des Lösungsmittels und Umkristallisieren z. B. in einem niederen Alkohol, wie Äthanol, wobei zur Erreichung einer besseren Ausbeute es vorteilhaft ist, wenn ein chlorwasserstoffhaltiges Lösungsmittel verwendet wird.

Die Ausgangsverbindung, das 4-Methyl-imidazol, kann in reiner Form oder in technischer Qualität verwendet werden.

Das erfindungsgemäße Verfahren der direkten Chlormethylierung eines Imidazols in 5-Stellung in glatter Reaktion ohne störende Nebenreaktionen und durchführbar in technischem Maßstabe ist überraschend. Eine derart einfache Chlormethylierung war nicht zu erwarten, da bei der Reaktion in einem stark sauren Medium das

Imidazol bekanntlich durch Protonierung desaktiviert wird. Auch war die hohe Selektivität der Einführung der Chlormethylgruppe in der 5-Stellung ohne störende Substitutionen in 1- oder 2-Stellung nicht zu erwarten, da sich beispielsweise am Stickstoffatom 1 ein normalerweise leicht ersetzbares Wasserstoffatom befindet. Vorteilhafterweise werden auch keine störenden Nebenreaktionen der erhaltenen 5-Chlormethylverbindung, z. B. Verknüpfung von Imidazolringen über $CH_2$-Gruppen, wie die Bildung von Diimidazolylmethan oder höherkondensierten Produkten, beobachtet.

Die Erfindung macht das 4-Methyl-5-chlormethylimidazol, das ein wichtiges Zwischenprodukt für weitere Imidazolderivate darstellt, erstmals technisch leicht zugänglich.

Es sei erwähnt, daß interessanterweise bisher direkte Chlormethylierungen an Imidazolen noch nicht beschrieben worden sind. Auch ist beispielsweise bei dem ähnlichen 3,5-Dimethyl-pyrazol mit einer freien NH-Gruppe eine eindeutige Chlormethylierung am Kohlenstoffatom 4 nicht möglich, da bei Raumtemperaturen vorwiegend Substitution am Stickstoffatom und bei erhöhten Temperaturen Polymerisationen eintreten, wie es von J. Dvoretzky und J. Richter im J. org. Chem. 15, 1285—1288 (1950) beschrieben wird.

Das erfindungsgemäße Verfahren soll durch die folgenden Beispiele beschrieben, aber nicht eingeschränkt werden.

Beispiel 1

Zu einer 80°C warmen Lösung von 167 Teilen technischem 4-Methyl-imidazol (92,6%ig) in 826 Teilen wäßriger konzentrierter Salzsäure (ca. 36 Gew.-%) tropft man unter gleichzeitigem Einleiten eines HCl-Gas-Stromes (ca. 65 Gewichtsteile pro Stunde, entsprechend 40 000 Volumenteile pro Stunde) innerhalb von 4 Stunden 218 Teile 37%ige Formaldehydlösung. Anschließend wird unter weiterem Einleiten von HCl-Gas noch 20 Stunden auf 80°C erwärmt. Die wäßrige Salzsäure wird nach Beendigung der Reaktion im Wasserstrahlvakuum weitgehend abdestilliert. Den Rückstand versetzt man mit 700 Teilen HCl-gashaltigem Äthanol ($\varrho = 0,88$) und erhitzt zum Sieden. Es wird auf ca. +5°C abgekühlt und der ausgefallene Niederschlag abgesaugt, mit HCl-gashaltigem Äther gewaschen und im Vakuum getrocknet. Es werden 197,5 Teile 4-Methyl-5-chlormethyl-imidazol-hydrochlorid vom Schmelzpunkt 213°C erhalten. Aus der Mutterlauge und der ätherischen Waschlösung werden weitere 17,4 Teile vom Schmelzpunkt 206°C erhalten, so daß die Gesamtausbeute 68,2% beträgt.

Beispiel 2

Ein Gemisch aus 124 Teilen wäßriger konzentrierter Salzsäure (ca. 36 Gew.-%), 25 Teilen 4-Methylimidazol (92,6%ig) und 10,8 Teilen Paraformaldehyd wird in einem geschlossenen Emaillekessel 16 Stunden auf 90°C erhitzt. Danach wird die Salzsäure im Wasserstrahlvakuum weitgehend abdestilliert und der Rückstand in 160 Teilen siedendem HCl-gashaltigen Äthanol ($\varrho = 0,88$) gelöst. Nach dem Abkühlen werden die ausgefallenen Kristalle abgesaugt, mit HCl-gashaltigem Äthanol und Äther gewaschen und im Vakuum getrocknet. Es werden 20 Teile vom Schmelzpunkt 214 bis 216°C erhalten. Aus der Mutterlauge und den Waschlösungen werden weitere 4,2 Teile vom Schmelzpunkt 209 und 210°C erhalten, so daß die Gesamtausbeute 51,3% beträgt.

## Patentansprüche

1. Verfahren zur Herstellung von 4-Methyl-5-chlormethyl-imidazol-hydrochlorid, dadurch gekennzeichnet, daß man 4-Methylimidazol in wäßriger Lösung mit Formaldehyd oder eines Oligomeren des Formaldehyds in Gegenwart einer überschüssigen Menge Chlorwasserstoff bei Temperaturen von 25 bis 160°C umsetzt.

2. Verfahren nach Anspruch 1, dadurch gekennzeichnet, daß man die Umsetzung in wäßriger Salzsäure bei gleichzeitigem Durchleiten von gasförmigem Chlorwasserstoff und bei einem Molverhältnis von 4-Methylimidazol zu Formaldehyd von 1 : 1 bis 1 : 1,5 durchführt.

3. Verfahren nach Anspruch 1, dadurch gekennzeichnet, daß man die Umsetzung in einem geschlossenen System unter Druck durchführt.

## Claims

1. A process for the preparation of 4-methyl-5-chloromethylimidazole hydrochloride, characterized in that 4-methylimidazole, in aqueous solution, is reacted with formaldehyde or a formaldehyde oligomer in the presence of an excess of hydrogen chloride, at from 25 to 160°C.

2. A process as claimed in claim 1, characterized in that the reaction is carried out in aqueous hydrochloric acid, whilst at the same time passing gaseous hydrogen chloride through the mixture, and using a molar ratio of 4-methylimidazole to formaldehyde of from 1 : 1 to 1 : 1.5

3. A process as claimed in claim 1, characterized in that the reaction is carried out in a closed system under pressure.

## Revendications

1. Procédé de préparation de chlorhydrate de 4-méthyl-5-chlorométhylimidazol, caractérisé par le fait qu'on fait réagir, à des températures de 25 à 160°C, du 4-méthylimidazol en solution aqueuse avec du formaldéhyde ou un oligomère du formaldéhyde en présence d'un excès d'acide chlorhydrique.

2. Procédé selon la revendication 1, dans lequel la réaction est effectuée dans de l'acide

chlorhydrique aqueux avec balayage simultané d'acide chlorhydrique gazeux et un rapport molaire entre le 4-méhylimidazol et le formaldéhyde compris entre 1/1 et 1/1,5.

3. Procédé selon la revendication 1, dans lequel la réaction est effectuée dans un système fermé sous pression.